# EUROPEAN PATENT APPLICATION

(11) **EP 4 574 142 A1**
(43) Date of publication of application: **25.06.2025**
(21) Application number: 23220197.0
(22) Date of filing: 23.12.2023
(51) Int. Cl.: A61K 31/047, A61K 31/51, A61K 31/724, A61K 36/185, A61K 47/00, A61P 25/28

(54) **COMPOSITION FOR USE IN ENHANCING, BOOSTING, RESTORING AND/OR PREVENTING THE DECLINE OF COGNITIVE FUNCTIONS**

(71) Applicant: Instytut Badawczy Innowacyjno Rozwojowy "Biotomed" Sp. Z O.O., 15-875 Bialystok (PL)
(72) Inventor: TOMULEWICZ, Mikolaj., 15-507 Bialystok (PL)
(74) Representative: Dalek, Arkadius Jan

(57) **Abstract**

The present invention concerns a composition for use in enhancing, boosting, restoring and/or preventing the decline of cognitive functions, an adjuvant composition and a use of kit-of-parts comprising the composition or adjuvant composition.

## Description

The present invention concerns a composition for use in enhancing, boosting, restoring and/or preventing the decline of cognitive functions, in detail according to the independent claims.

Alzheimer's disease (AD) is recognized by the World Health Organization (WHO) as a global priority for public health and poses a serious challenge to the health and social care system. It is a decline of cognitive functions. Despite the significant increase of knowledge about the pathogenesis of AD and how the disease has been conceptualized since Alois Alzheimer described her first case in 1907, there are still no known ways of treating or preventing it. Nowadays and with the current state of knowledge it is an incurable disease. Alzheimer's disease is the cause of dementia, accounting for 50-70% of all cases. Symptoms of Alzheimer's include thinking disorder, speech disorder, and getting lost. Dementia itself as an acquired progressive cognitive impairment sufficient to influence daily activities - is a major cause of addiction, disability and mortality. Current estimates indicate that today there are almost 44 million people with Alzheimer's disease in the world, and another new case is diagnosed every 4 seconds. According to WHO data, by 2030 this number will increase to 65 million, and in 2050 to 115 million. It is estimated that in Poland the population of people over 65 is currently about 14.7%, in 2035 it will increase to 24.5%, and in 2050 it will reach over 30%. According to data from the Central Statistical Office in Poland, there are currently over 300,000 people with Alzheimer's disease, and by 2050 this number will triple and amount to almost 1 million patients. Most cases of Alzheimer's disease appear spontaneously.

Alzheimer's disease is a chronic neurodegenerative disorder characterized by progressive loss of memory and cognitive functions. In people with developing Alzheimer's disease, increasing learning and memory impairment after all leads to the development of a full-blown disease. In a small percentage, problems with language, executive functions, perception (agnosia) or movement (apraxia) are more important than problems with memory.

The cause of most cases of Alzheimer's disease is unknown, with the exception of 1-5% of cases where the disease was genetic. β-amyloid deposition and neurofibrillary tangles, the main neuropathological features of Alzheimer's disease, occur in selectively sensitive areas of the brain, such as the hippocampus and forebrain cortex.

There are several competing hypotheses trying to explain the cause of the disease. Based on these hypotheses, so far few drugs for Alzheimer's disease have been proposed, and moreover their effectiveness is not high enough.

Among the many factors listed as responsible for Alzheimer's disease, the most important seem to be genetic factors, aggregation of β-amyloid and tau protein.

Extracellular deposits of β-amyloid are the primary cause of the disease, which leads to disturbances in interneuronal communication, resulting in the formation of neurotoxins and, consequently, leading to the death of neurons.

β-amyloid, the main component of amyloid plaques, is produced from the amyloid precursor protein (Amyloid Precursor Protein, APP) through a series of protease cleavages, using enzymes called secretases. As a result of this process, N-terminal, shorter fragments are formed, which perform various, important, but not yet recognized functions. The gene encoding the APP protein is mutated by the same type of enzyme that allows HIV infection.

Based on the above hypothesis, it was proposed to introduce some antiretroviral drugs for treatment. Indeed, it was found that the experimental vaccine against the fragment of APP, Aβ1-42 protein removes amyloid plaques, but did not have a significant effect on dementia [Holmes et al., 2008]. The assessment of verubecestat, which inhibits β-secretase 1, responsible for the formation of β-amyloid, was discontinued because no positive clinical effect was observed [Egan et al., 2018].

The search for drugs for the treatment of Alzheimer's disease is still a big problem because there is no specific goal to fight the disease. From many substances, natural compounds as potential drugs have also been studied. Preclinical studies have provided very promising results, matching or surpassing the potency and effect of drugs used in standard clinics.

From the Chinese patent CN 106265793, the use of an extract of the mangrove plant Acanthus ilicifolius L is known for the preparation of drugs for the treatment and/or prevention of Alzheimer's disease.

From the international patent WO/2018/133563, plant extract of Panax and its pharmaceutical composition are known for to make a medicine for chronic heart failure, diabetes or Alzheimer's disease. On the other hand, another international patent WO/2018/096039 discloses a method for producing an extract of Tagetes L (marigold) enriched with leontopodic acid B and a method for producing a pharmaceutical composition serving in particular as a medicine for the prevention and/or treatment of neurodegenerative disease, in particular Alzheimer's disease.

From the European patent EP 2712619 a pharmaceutical composition is known containing extracts from the species Monsonia sp. for the prevention and/or treatment of dementia with an excellent β-amyloid inhibitory effect, which is known as an Alzheimer's causative agent.

It is therefore the object of the present invention to provide a composition for use in enhancing, boosting, restoring and/or preventing the decline of cognitive functions that is improved as compared to the devices or methods known from the state of the art. Also, the present invention shall provide a promising, new and effective adjuvant therapy to use in combination with subeffective doses anti-AD medications.

The object is solved by a composition for use in enhancing, boosting, restoring and/or preventing the decline of cognitive functions according to the independent claims. The dependent claims describe particularly useful embodiments of the invention.

It has been found that pentacyclic triterpenes have neuroprotective properties, which can be extremely useful in preventing and treating Alzheimer's disease, in detail when combined with cyclodextrin.

Pentacyclic triterpenes such as betulin can be represented by the following formula: 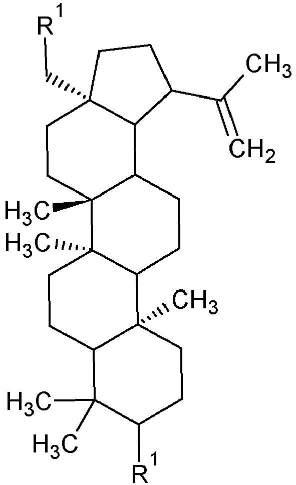 wherein the R¹ substituent is a -OH (betulin) group which can have the cyclodextrin.

According to the invention, a composition for use in the treatment, enhancing, boosting, restoring and/or preventing the decline of cognitive functions in a subject in need, especially a human or an animal, said composition comprising:
- from 100 mg to 250 mg, especially from 100,00 mg to 250,00 mg of betulin; and
- from 580 mg to 1465 mg, especially from 580,00 mg to 1465,00 mg, more especially from 585,94 mg to 1464,85 mg of γ (gamma)-cyclodextrin.

That means, that the ratio of betulin to γ (gamma)-cyclodextrin may be between 0,16 and 0,18, especially may amount 0,17, especially 0,171, 0,172, 0,173, 0,174, 0,175, 0,176, 0,177, 0,178 or 0,179.

Among natural cyclodextrins, γ-cyclodextrin is best soluble in water, while β-cyclodextrin is the least. The solubility of γ-cyclodextrin is 232 mg/mL, at 25°C, while that of β-cyclodextrin is only 18.5 mg/mL, at 25°C.

Orally administered cyclodextrins are non-toxic at low to moderate doses due to lack of absorption from the gastrointestinal tract. Due to nephrotoxicity and lower solubility β-cyclodextrin cannot be administered parenterally.

γ-cyclodextrin is easily digested in the small intestine, while both α- and β-cyclodextrin are digested much more poorly. All parent cyclodextrins are accepted as food additives and "generally recognized as safe" (GRAS). As dietary supplement the total daily oral dose of α-cyclodextrin may reach 6000 mg/day, for β-cyclodextrin 500 mg/day and for γ-cyclodextrin 10 000 mg/day, and for HP-β-cyclodextrin as oral pharmaceutical 8000 mg/day.

In terms of safety of use, to α-cyclodextrin, β-cyclodextrin and its derivative, RM-β-cyclodextrin showed renal toxicity at relatively low doses after parenteral administration and thus are not suitable for medicinal products given intravenously. High doses of ≥600 mg/kg of γ-cyclodextrin showed only reversible vacuolation in the renal tubular epithelium of rats.

In contrast, the acceptable oral intake of cyclodextrins is defined by the No Observed Adverse Effect Level (NOAEL). This means the highest concentration or amount of a substance at which there are no detectable adverse effects in the exposed population. For β-cyclodextrin, the NOAEL is 10 mg/kg/day, while for γ-cyclodextrin, it is 10 times higher at 100 mg/kg/day.

The composition may comprise from of 111,12 mg to 277,8 mg of an extract, especially purified dry extract from birch bark of Betula pendula, Betula pendula Roth, Betula pubescens, as well as hybrids of these species as equivalent to 0,65 to 1,653 g birch bark, corresponding to 100 mg to 250 mg, especially from 100,00 mg to 250,00 mg betulin.

Advantageously, the composition may comprise further from 0,33 mg to 0,66 mg of thiamine.

Cytotoxicity studies using the XTT assay showed that betulin (1-30 µM) reduced the viability of differentiated SH-SY5Y neuroblastoma cells in a dose-dependent manner and induced a cytotoxic effect (viability below the 70% threshold) above a concentration of 20 µM. In contrast, addition of thiamine increased the survival of SH-SY5Y differentiated neuroblastoma cells over the entire range of concentrations tested (1 - 30 µM).

Furthermore, a statistically significant protective effect of betulin against differentiated SH-SY5Y cells was observed for concentrations of 1, 2, 5 and 10 µM. The increase in cell viability relative to the EC50 value for the positive control ranged from 12.2 to 15.2%. In contrast, at concentrations of 15 and 20 µM, betulin showed no protective effect and further enhanced the toxic effect of hydrogen peroxide. When thiamine was added, a statistically significant protective effect against differentiated neuroblastoma cells for a toxin concentration equal to EC50 was observed for the entire range of concentrations used, i.e. 1-20 µM. Higher concentrations of the composition, i.e. 20, 15 and 10 µM, improved cell viability by 26.1%-22.5%, while lower concentrations, i.e. 5, 2 and 1, ranged from 19.3 to 16.7%.

Thereby, the amount of the composition or dosage form, administered to the subject in need, especially a human or an animal, may be in the range of 100 to 1000, especially in the range of 100,00 to 1000,00 mg/kg body weight/day, especially from 250 to 500, more especially from 250,00 to 500,00 mg/kg body weight/day.

In detail, 250 mg to 500 mg, especially 250,00 mg to 500,00 mg of the composition may be administered once per day.

The composition may be a pharmaceutical and/or dietary supplement composition comprising an effective amount of the composition.

Further, the decline of cognitive functions may be at least one of the dementia syndrome and the early stages and pre-stages.

Thereby, the dementia syndrome may be selected from light dementia, moderate dementia, Alzheimer dementia, vascular dementia and their mixed forms.

The early stages and pre-stages of dementia may be light cognitive impairments (MCI), cognitive impairments, which are not dementia yet (CIND), subjectively perceived impairments of the memory, subjective impairments of other cognitive components of performance, objectively impaired memory performance, objective impairments of other cognitive components of performance, depressive disorder, anxiety, frightened mood, apathy, lack of motivation, indifference, irritability, excitement, sleeping disorders, and disorders of day and night rhythm.

The present invention also concerns an adjuvant composition for use in enhancing, boosting, restoring and/or preventing the decline of cognitive functions in a subject in need, said adjuvant composition comprising a composition as described in any one of the claims, wherein said adjuvant composition is to be administered concurrently, intercurrently, consecutively or concomitantly with at least one of the dementia syndrome and the early stages and pre-stages of dementia or Alzheimer's disease medication, preferably in a subeffective dose.

Also, the present invention concerns a use of a composition or adjuvant composition as described in any one of the claims, as a non-therapeutic dietary supplement for enhancing, boosting and/or restoring memory or learning.

Furthermore, the present invention concerns a use of a kit-of-parts, comprising the composition or adjuvant composition as described in any one of the foregoing claims as a non-therapeutic dietary supplement for enhancing, boosting and/or restoring memory or learning.

### Experimental section

### Investigation of betulin neuroprotective mechanisms

The study was conducted on Wistar rats weighing 220-250 g. All animals were subjected to two weeks of observation prior to the experiment to exclude infection. Rats were housed in standard cages, 5 individuals in each. The animals stayed in a room with a temperature of 22 ± 2 ° C, air humidity 60 - 70% and in natural lighting (12/12 in a day / night cycle). The animals had free access to water and standard laboratory feed *ad libitum.*

The Alzheimer's disease model was induced by administering aluminum chloride AlCl₃ (200 mg/kg body weight, i.g.) to the animals for 120 days. Aluminium is highly neurotoxic, and chronic exposure to AlClₐ causes a significant increase in the activity of acetylcholinesters, expression of the precursor protein amyloid, TNFα [Rodella et al., 2008; Rather et al., 2018].

Animals were assigned to groups: control group receiving saline (sham operation), group with AlCl₃ without treatment and group with AlCl₃ receiving betulin (100 mg/kg/day, i.g., within the last 50% of the days of the experiment) and betulin in the complex with cyclodextrin (100 mg/kg/day, i.g., in the last 50% of the days of the experiment). Betulin or betulin in a complex with cyclodextrin was administered orally with an intragastric probe in a 2% starch emulsion, reflecting the composition of the finished preparation given in the examples of use.

On the last day of the experiment, the animals were anaesthetized. Brains were quickly collected and then divided into different areas. The entire procedure was carried out in a cold room (+4°C) on ice, and the samples were frozen in liquid nitrogen and stored in an ultra-low temperature refrigerator for further analysis.

In order to measure rat brain damage caused by aluminum chloride, biochemical determinations were carried out on the collected brain tissue fragments.

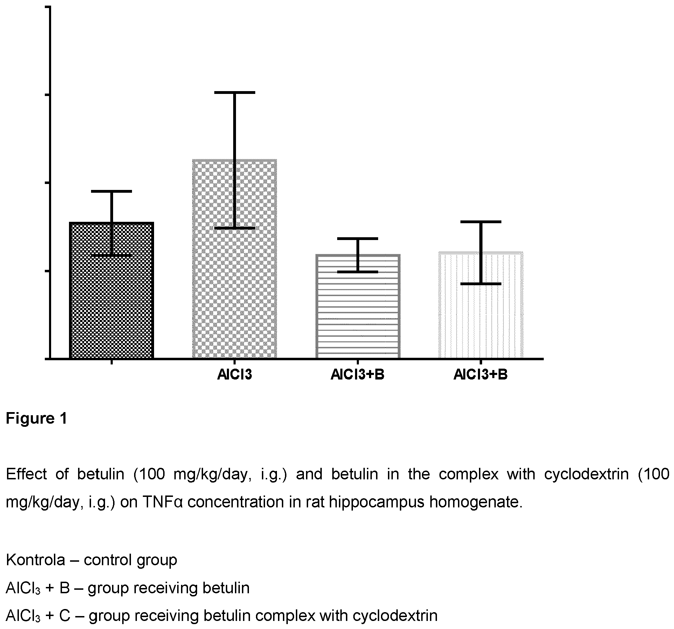

The obtained test results are given in Table 1 and in Figure 1. Neuroprotective effects were shown in all treatment groups administered betulin (100 mg/kg/day, i.g.) or betulin in the complex with cyclodextrin (100 mg/kg/day, i.g.). Betulin reduced the concentration of TNFα in the hippocampal homogenates of the brains of the animals studied. Much better effects were observed in the group given betulin in the comex with cyclodextrin.

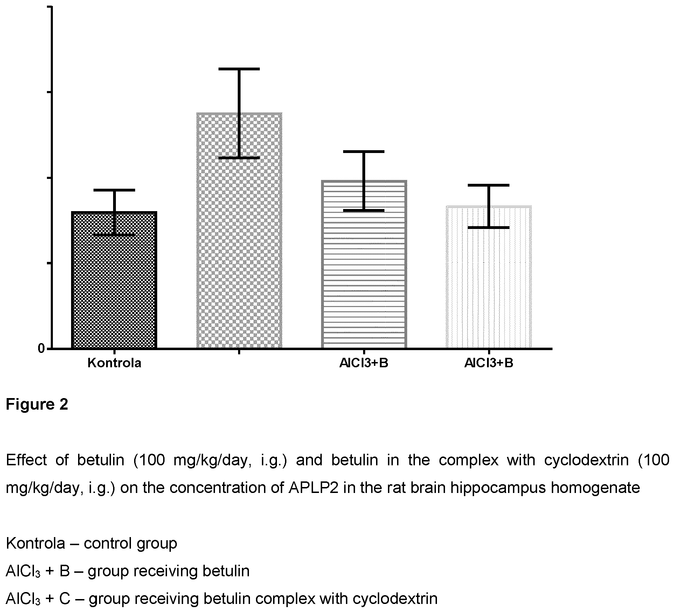

Similarly, Table 2 and Figure 2 present the results of studies on the effect of betulin on the concentration of the precursor protein APLP2. Neuroprotective effects have been demonstrated in all treatment groups given betulin (100 mg/kg/day, i.g.) or betulin in the complex with cyclodextrin (100 mg/kg/day, i.g.). Betulin reduced the concentration of APLP2 in the hippocampal homogenates of the brains of the animals studied. As in the previous case, much better effects were observed in the group to which betulin was administered in combination with cyclodextrin.

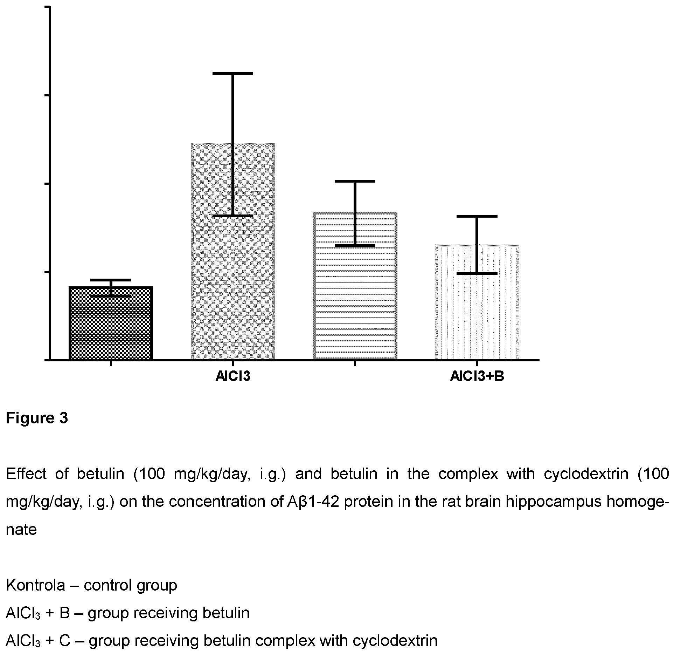

Table 3 and Figure 3 present the results of studies on the effect of betulin on the concentration of β-amyloid. Neuroprotective effects have been demonstrated in all treatment groups given betulin (100 mg/kg/day, i.g.) or betulin in the complex with cyclodextrin (100 mg/kg/day, i.g.). Betulin reduced the concentration of β-amyloid in the hippocampal homogenates of the brains of the animals studied. Also in this case, much better effects were observed in the group to which betulin was administered in a complex with cyclodextrin.

**Table 1. Effect of betulin (100 mg/kg/day, i.g.) and betulin complex with cyclodextrin (100 mg/kg/day, i.g.) on TNFα concentration in rat hippocampal homogenate.**

| Parameter | Control | AlCl₃ | AlCl₃+B | AlCl₃+C |
|---|---|---|---|---|
| TNF-α, pg/mg | 7.70±0.65 | 11.30±1.46 ^{a} | 5.88±0.39 ^{b} | 6.03±0.67 ^{b} |

| | | | | |
|---|---|---|---|---|
| ^{a} - p<0.05 compared to the control group, ^{b} - p<0.05 compared to the AlCl₃ group AlCl₃ + B - group receiving betulin AlCl₃ + C - group receiving betulin complex with cyclodextrin | | | | |

**Table 2. Effect of betulin (100 mg/kg/day, i.g.) and betulin in the complex with cyclodextrin (100 mg/kg/day, i.g.) on the concentration of APLP2 in the rat brain hippocampus homogenate**

| Parameter | Control | AlCl₃ | AlCl₃+B | AlCl₃+C |
|---|---|---|---|---|
| APLP2, pg/100 mg | 477,9±24,9 | 824,8±55,1^{a} | 588,1±36,4^{ab} | 499,2±26,3^{b} |

| | | | | |
|---|---|---|---|---|
| ^{a} - p<0.05 compared to the control group, ^{b} - p<0.05 compared to the AlCl₃ group AlCl₃ + B - group receiving betulin AlCl₃ + C - group receiving betulin complex with cyclodextrin | | | | |

**Table 3. Effect of betulin (100 mg/kg/day, i.g.) and betulin in the complex with cyclodextrin (100 mg/kg/day, i.g.) on the concentration of Aβ1-42 protein in rat hippocampal homogenate**

| Parameter | Control | AlCl₃ | AlCl₃+B | AlCl₃+C |
|---|---|---|---|---|
| Protein Aβ₁₋₄₂, pg/100 mg | 40,92±1,56 | 121,90±14,30^{a} | 83,25±6,40^{ab} | 65,34±5,70^{b} |

| | | | | |
|---|---|---|---|---|
| ^{a} - p<0.05 compared to the control group, ^{b} - p<0.05 compared to the AlCl₃ group AlCl₃ + B - group receiving betulin AlCl₃ + C - group receiving betulin complex with cyclodextrin | | | | |

### Influence of betulin on spatial learning and memory in Morris water maze (MWM) test and a motor coordination in beam walking test in rats with Alzheimer-like neurodegeneration (last testing, 5th day of testing)

The results MWM tests shows that time to reach the platform in the rats as an animal model of AD increased significantly and differed significantly from the results of other animals. The time spent in a target quadrant in rats from the second group was considerably low. The rats from group 2 demonstrated a two-fold increase in both the latency to initiate the task and the total time to cross the beam (p < 0.05). Per cent of successful trials was only 29% vs 100% in the remaining groups.

**Table 4. Results of the Morris water maze test and motor coordination in beam walking test in rats in the experiment**

| Groups | Morris water maze test | | Beam walking test | |
|---|---|---|---|---|
| | Parameter | | | |
| | Time to reach the platform, seconds | Time spent in a target quadrant. seconds | Time to reach the shelter, seconds | Per cent of successful trials |
| Control | 31±6 | 41±4 | 13.1±2.6 | 100 |
| AlCl₃ | 51±8^{a} | 19±6^{a} | 26.1±5.9^{a} | 29^{a} |
| AlCl₃ + B | 38±7^{b} | 26±8^{ab} | 18.6±3.6^{ab} | 100^{b} |
| AlCl₃ + C | 33±8^{b} | 33±5^{b} | 15.6±3.3^{b} | 100^{b} |

| | | | | |
|---|---|---|---|---|
| ^{a} - *p*<0.05 compared to the control group, ^{b} - *p*<0.05 compared to the AlCl₃ group AlCl₃ + B - group receiving betulin AlCl₃ + C - group receiving betulin complex with cyclodextrin | | | | |

### Influence of betulin on acetylcholine esterase activity in brain hemispheres and hippocampus in rats with Alzheimer-like neurodegeneration

One of the treatment method of AD focuses on normalising cholinergic neurotransmission in the brain. The efficacy of acetylcholinesterase inhibitors is explained by acetylcholine's stimulation of muscarinic M1 receptors, which, by activating protein kinase C, increase α-secretase activity and thus prevent the formation of senile plaques. In the AlCl₃-induced AD model animals, statistically significantly reduced enzyme activity was observed compared to healthy control animals in both the hippocampus and cerebral hemispheres. From this results, a hypothesis can be drawn that betulin may be an acetylcholinesterase inhibitor, as administration of betulin resulted in a statistically significant increase in acetylcholinesterase activity (p<0.05) in both brain hemispheres and hippocampus to values compared in a control group of healthy animals.

**Table 5. Acetylcholine esterase activity in brain hemispheres and hippocampus of rats with AlCl₃-intoxication (200 mg/kg/day of BW, i.g., 45 days) treated with betulin, µmol/h/mg protein**

| Groups | Brain hemispheres | Hippocampus |
|---|---|---|
| Control | 16.95 ± 0.67 | 19.36 ± 0.61 |
| AlCl₃ | 9.76 ± 0.54^{a} | 8.66 ± 0.63^{a} |
| AlCl₃ + B | 16.20 ± 0.55^{b} | 14.22 ± 0.84^{b} |

| | | |
|---|---|---|
| ^{a} - p<0.05 compared to the control group, ^{b} - p<0.05 compared to the AlCl₃ group AlCl₃ + B - group receiving betulin | | |

### Influence of betulin on oxidative stress parameters in rats with Alzheimer-like neurodegeneration

Oxidative stress is considered one of the causes of AD. Hydroxyl radical inhibits the activity of monoamine oxidases in the brain, which are responsible for the catabolism of neurotransmitters. It also contributes to neuronal loss in brain ischaemia situations in AD. The body has developed specific defence mechanisms to prevent damage caused by ROS.The first line of defence includes the following antioxidant enzymes: superoxide dismutases (SODs), catalase (CAT), glutathione peroxidases (GPxs), glutathione reductase (GR) and glutathione S-transferases (GSTs).

A reduction in GSH, GR and GSTs activity was noted in animals with induced AD. Administration of betulin resulted in a significant increase in the activity of all enzymes. In addition, an increase in GSSG, G6PD and GPO activity was observed in animals with induced AD. Again, administration of betulin reversed this effect and caused a significant decrease in the activity of these enzymes.

**Table 6. Glutathione content and some antioxidant enzymes activities in brain hemispheres during Al-intoxication and after treatment with betulin**

| Groups | GSH, nmol/mg protein | GSSG, nmol/mg protein | G6PD, nmol NADPH/min/ mg protein | Glutathione reductase, nmol NADPH/min/ mg protein | Glutathione-S-transferase, nmol CDNB/min/mg protein | GPO, umol GSH/mi n/mg protein |
|---|---|---|---|---|---|---|
| Control | 28.45±1.25 | 0.11±0.002 | 29.42±1.80 | 11.78±1.27 | 146.90±14.83 | 36.21 ± 3.02 |
| AlCl₃ | 21.16±1.40^{a} | 0.13±0.003^{a} | 34.13±1.42^{a} | 10.61±1.75 | 88.63±9.39^{a} | 65.92 ± 2.41^{a} |
| AlCl₃ + B | 26.09±2.19^{b} | 0.112±0.002^{b} | 28.61±1.62^{b} | 11.59±1.39 | 129.90±10.45^{b} | 33.63 ± 2.09^{b} |

| | | | | | | |
|---|---|---|---|---|---|---|
| ^{a} - p<0.05 compared to the control group, ^{b} - p<0.05 compared to the AlCl₃ group AlCl₃ + B - group receiving betulin | | | | | | |

The test results of Table 6 showing that the invention provides the advantages achieved by such a composition in treatment, enhancing, boosting, restoring and/or preventing the decline in cognitive functions in a subject in need, such like AD.

Pharmaceutical compositions with neuroprotective effect used in the prevention and treatment of Alzheimer's disease are prepared and administered in various forms. It is obvious to those skilled in the art that unit doses may contain as active ingredient one or more of the compounds described. It is also evident that both dosages and methods of administration will vary depending on the needs of the patient and depending on the particular activity of the active ingredient (s). Determining the dosage and method of administration remains within the skill of each practicing physician.

## Claims

1. Composition for use in the treatment, enhancing, boosting, restoring and/or preventing the decline of cognitive functions in a subject in need, said composition comprising:
- from 100 mg to 250 mg, especially from 100,00 mg to 250,00 mg of betulin; and
- from 580 mg to 1465 mg, especially from 580,00 mg to 1465,00 mg, more especially from 585,94 mg to 1464,85 mg of γ (gamma)-cyclodextrin.

2. Composition according to claim 1, wherein the composition comprises from of 111,12 mg to 277,8 mg of an extract, especially purified dry extract from birch bark of Betula pendula, Betula pendula Roth, Betula pubescens, as well as hybrids of these species as equivalent to 0,65 to 1,653 g birch bark, corresponding from 100 mg to 250 mg, especially from 100,00 mg to 250,00 mg betulin.

3. Composition according to claim 1 or 2, wherein the composition comprises further comprises from 0,33 mg to 0,66 mg of thiamine.

4. Composition according to any one of the foregoing claims, the amount of the composition or dosage form, administered to the subject in need, is in the range of 100 to 1000, especially in the range of 100,00 to 1000,00 mg/kg body weight/day, more especially from 250 to 500, most especially from 250,00 to 500,00 mg/kg body weight/day.

5. Composition according to claim 4, wherein 250 mg to 500 mg, especially 250,00 mg to 500,00 mg of the composition is administered once per day.

6. Composition according to any one of the foregoing claims, wherein the composition is a pharmaceutical and/or dietary supplement composition comprising an effective amount of the composition.

7. Composition according to any one of the foregoing claims, wherein the decline of cognitive functions is at least one of the dementia syndrome and the early stages and pre-stages.

8. Composition according to claim 7, wherein the dementia syndrome is selected from light dementia, moderate dementia, Alzheimer dementia, vascular dementia and their mixed forms.

9. Composition according to claim 7, wherein the early stages and pre-stages of dementia are light cognitive impairments (MCI), cognitive impairments, which are not dementia yet (CIND), subjectively perceived impairments of the memory, subjective impairments of other cognitive components of performance, objectively impaired memory performance, objective impairments of other cognitive components of performance, depressive disorder, anxiety, frightened mood, apathy, lack of motivation, indifference, irritability, excitement, sleeping disorders, and disorders of day and night rhythm.

10. Adjuvant composition for use in enhancing, boosting, restoring and/or preventing the decline of cognitive functions in a subject in need, said adjuvant composition comprising a composition as described in any one of the foregoing claims, wherein said adjuvant composition is to be administered concurrently, intercurrently, consecutively or concomitantly with at least one of the dementia syndrome and the early stages and pre-stages of dementia or Alzheimer's disease medication, preferably in a subeffective dose.

11. Use of a composition or adjuvant composition as described in any one of the foregoing claims, as a non-therapeutic dietary supplement for enhancing, boosting and/or restoring memory or learning.

12. Use of a kit-of-parts, comprising the composition or adjuvant composition as described in any one of the foregoing claims as a non-therapeutic dietary supplement for enhancing, boosting and/or restoring memory or learning.
